# EUROPÄISCHE PATENTANMELDUNG

(11) **EP 2 338 558 A1**
(43) Veröffentlichungstag der Anmeldung: **29.06.2011**
(21) Anmeldenummer: 10192876.0
(22) Anmeldetag: 29.11.2010
(51) Int. Cl.: A61N 1/08, A61N 1/39

(54) **Detektor für elektromagnetische Felder**

(30) Priorität: 22.12.2009 US 288853 P
(71) Anmelder: Biotronik CRM Patent AG, 6341 Baar (CH)
(72) Erfinder: Dörr, Thomas, 12437, Berlin (DE)
(74) Vertreter: Lindner-Vogt, Karin L.

(57) **Zusammenfassung**

Batteriebetriebenes elektrisches Gerät und/oder implantierbares medizinisches Gerät, wobei das Gerät mindestens beinhaltet:
eine Batteriespannungsüberwachungseinheit, die ausgestaltet ist einen Spannungsverlauf an einer Batterie während des Ladens eines Kondensators über einen Transformator in einer Ladeschaltung zu überwachen und anhand der Spannungscharakteristik eine magnetische Vorsättigung des Transformators bestimmen kann und eine Kontrolleinheit, die den Ladevorgang immer dann abbricht, wenn ein vordefinierter Schwellwert für die magnetische Vorsättigung des besagten Transformators überschritten wird.

## Beschreibung

### Gebiet der Erfindung

Die Erfindung bezieht sich auf ein Verfahren zur Erkennung von elektromagnetischen Feldern, speziell auf solche, wie sie bei bildgebenden Kernspintomographie- (MRI-, MRT-) Geräten auftreten, für batteriebetriebene Geräte, wie implantierbare medizinische Geräte.

### Stand der Technik

Obwohl MRI-Untersuchungen einen immer höheren Stellenwert in der diagnostischen Medizin erhalten, ist ein Teil der Patienten für MRI-Untersuchungen kontraindiziert. Eine solche Kontraindizierung kann durch ein aktives implantiertes medizinisches Gerät gegeben sein.

Problematisch sind neben möglichen Erwärmungseffekten von Implantaten, besonders bei kleinen leitenden Strukturen, die hier nicht adressiert werden sollen, fehlerhafte Erkennungen von Ereignissen im Herzen, wie aber nicht ausschließlich Kammerflimmern, bzw. schnelle Herzrhythmusstörungen, (VF), und die hohen statischen Magnetfelder und daraus resultierende Magnetisierungen von elektrischen Komponenten, besonders bei aktiven Implantaten wie aber nicht ausschließlich Defibrillatoren/Kardioverter, im Weiteren ICD. Aber auch andere batteriebetriebene Geräte können durch elektromagnetische Felder in Ihrer Funktion gestört werden und dürfen nicht in Umgebungen mit erhöhten elektromagnetischen Belastungen betrieben werden. Der folgende Stand der Technik geht vor allem auf die Problematik bei implantierten medizinischen Geräten (IMD) ein.

Die US 6,522,920 beschreibt ein System zum Schutz der induktiv angesteuerten Hochspannungsschalter eines ICD bei der Schockabgabe in einem Magnetfeld. Dazu wird überwacht, ob eine ausreichende Gatespannung zum Zeitpunkt der Schockabgabe bereit steht. Die Überwachung wird durch eine Beurteilung der Sekundärspannung der induktiven Treiber beurteilt.

Aus der US 2008/0071168 ist bekannt, zur Detektion eines Magnetfeldes eine Impedanzmesseinheit und ein RLC-Glied vorzusehen und die Detektion durch Bestimmung der Induktivität in der RLC-Komponente vorzunehmen. Dafür wird eine zusätzliche oder angepasste Impedanzmesseinheit benötigt.

Des Weiteren beschreibt die US 7,509,167 die Erkennung eines Magnetfeldes durch die Messung der Steuerzeiten für die Ansteuerung des Hochspannungstransformators der Primärseite oder alternativ die Messung von Spitzenströmen während eines Ladezyklus. Nachteil des beschriebenen Systems ist, dass die Messung der Steuerzeiten sehr schnell sein muss (µs bis ns), bzw. dass eine direkte Strommessung aufgrund des notwendigen Messwiderstandes, der die Ladezeiten für den Hochspannungskondensator verlängern würde, nur schwer implementierbar ist.

Daher ist es Aufgabe der Erfindung, eine einfach zu implementierende Magnetfelderkennung, spezielle eine MRI-Erkennung bereitzustellen.

Die Aufgabe wird durch ein batteriebetriebenes elektrisches Gerät (BED) mit den Merkmalen der Anspruchs 1 gelöst.

Das BED umfasst mindestens eine Batteriespannungsüberwachungseinheit 230, die ausgestaltet ist einen Spannungsverlauf 300, 300' an einer Batterie während des Ladens eines Kondensators über einen Transformator in einer Ladeschaltung 250 zu überwachen und anhand des Spannungsverlaufs eine magnetische Vorsättigung des Transformators zu bestimmen und eine Kontrolleinheit 220, die den Ladevorgang immer dann abbricht, wenn ein vordefinierter Schwellwert für die magnetische Vorsättigung des Transformators überschritten wird.

In einer bevorzugten Ausgestaltung erfolgt die Erkennung der Überschreitung des Schwellwerts für die magnetische Vorsättigung des Transformators über eine Messung einer minimalen Batteriespannung während des Ladens.

Ebenfalls bevorzugt erfolgt die Erkennung der Überschreitung des Schwellwerts für die magnetische Vorsättigung des Transformators über eine Messung eines maximalen Spannungsgradienten dU/dt am Ladebeginn.

Unter Ladebeginn sind die ersten Sekunden, bevorzugt die ersten 200 ms, besonders bevorzugt die ersten 100 ms und ebenfalls besonders bevorzugt die ersten 10 ms eines Ladevorganges, zu verstehen.

In einer weiteren Ausgestaltung erfolgt die Erkennung einer Überschreitung der Schwelle für die magnetische Vorsättigung des Transformators über eine Messung einer maximalen Spannungsdifferenz vor und während des Ladens.

Bei einer weiteren bevorzugten Ausgestaltung erfolgt die Erkennung einer Überschreitung der Schwelle für die magnetische Vorsättigung des Transformators über Messungen einer minimalen Batteriespannung während des Ladens und einer maximalen Spannungsdifferenz vor und während des Ladens.

Des Weiteren ist es bevorzugt, dass der Ladestrom derart begrenzt wird, dass eine minimale Batteriespannung nicht unterschritten wird.

In einer weiteren Ausgestaltung wird bevorzugt, dass bei Abbruch des Ladevorgangs die Ladeschaltung für eine vorgegebene Zeit deaktiviert wird.

Beispielsweise kann die Kontrolleinheit 220 den Ladevorgang aufgrund des Erreichens oder Überschreitens des Schwellwertes für die Vorsättigung des Transformators abbrechen.

Auch wird bevorzugt, dass die vorgegebene Zeit für die Deaktivierung der Ladeschaltung 250 bei aufeinander folgenden Ladeabbrüchen innerhalb einer zweiten vorgegebenen Zeit durch die Kontrolleinheit 220 und die Batteriespannungsüberwachungseinheit 230 jeweils um eine dritte vorgegebene Zeit verkürzt wird, wobei eine vorgegebene minimale Zeit für die Deaktivierung der Ladeschaltung nicht unterschritten wird und die erste Zeit zur Deaktivierung der Ladeschaltung 250 nach einer vierten vorgegebenen Zeit wieder hergestellt wird.

Bevorzugt ist das Gerät durch Programmierung oder Gerät durch Femprogrammierung, "remote-programming", in einen MRI-sicheren Zustand versetzbar.

Auch bevorzugt ist, dass beim Erkennen einer Überschreitung des Schwellwerts für die magnetische Vorsättigung des Transformators dies in einem Diagnostikspeicher notieren vermerkt wird und/oder während einer MRT-Untersuchung keine Episoden aufgezeichnet werden. So kann verhindert werden, dass eine MRT-Sitzung die Diagnostikdaten mit Artefakten überschreibt.

Unter Femprogrammierung ist dabei die Programmierung eines IMD zu verstehen, wobei das IMD und die Programmiereinheit, bzw. der Programmierende räumlich so von einander getrennt sind, dass die normalerweise vorhandene Nahfeld-Telemetrie (< 10 m) alleine nicht ausreicht, die räumliche Trennung zu überbrücken.

Besonders bevorzugt ist, dass die Kontrolleinheit 220 den Ladevorgang bei Erreichen oder Überschreiten des Schwellwertes für die Vorsättigung des Transformators abbricht und das batteriebetriebene elektrische Gerät für einen vorbestimmbaren Zeitraum in den MRI-sicheren Zustand versetzt.

In einer weiteren Ausführung wird ein Einschalten, oder das Eingeschaltet sein des MRI-sicheren Zustands mittels telemetrischer Femüberwachung, einem so genannten"Home Monitoring" übertragen. Das heißt, dass beim Einschalten des MRI-sicheren Zustandes ein Signal oder eine Information direkt oder über mindestens ein Zwischengerät, z. B. ein Patientengerät, an eine zentrale Einheit gesendet wird, die die Information oder das Signal bearbeiten oder gegebenenfalls weiterleiten. Sollte beim Einschalten des MRI-sicheren Zustands keine Verbindung zur zentralen Einheit bestehen oder aufbaubar sein, so wird die Information oder das Signal zu einem späteren Zeitpunkt gesendet oder übertragen, bevorzugt, sobald eine Verbindung zur zentralen Einheit besteht oder diese herstellbar ist. Telemetrische Femüberwachung bezeichnet dabei allgemein die Möglichkeit, dass Informationen oder Signale von einem IMD zu einer räumlich entfernten Einheit übertragen werden können, um eine Überwachung des Patienten auch ohne Arztbesuch oder Hospitalisierung zu ermöglichen, indem z. B. Daten aus dem Implantat über ein Service Center an einen zuständigen Arzt übermittelt werden.

Bevorzugt wird, dass das batteriebetriebene elektrische Gerät ein implantierbares medizinisches Gerät (IMD) ist.

Besonders bevorzugt wird, dass das IMD ein implantierbarer Defibrillator/Kardioverter (ICD) oder ein IMD zur kardialen Resynchronisation mit einem ICD ist.

Besonders bevorzugt wird, dass ein MRI-sicherer Zustand das Unterdrücken der Abgabe von Hochspannungsschocks umfasst.

Ebenfalls besonders bevorzugt wird, dass ein MRI-sicherer Zustand ein Unterdrücken der Abgabe von Schrittmacherstimulationen enthält oder aber eine Abgabe von asynchronen Schrittmacherimpulsen umfasst. Die Entscheidung über den geeigneten MRI-sicheren Zustand kann entweder beim Programmieren der des IMD oder automatisch beim Programmieren des IMD oder automatisch bei der Detektion eines MRI-Feldes oder automatisch zu vorbestimmten Zeitpunkten oder bei bestimmten Ereignissen oder Patientenzuständen getroffen werden.

Ebenfalls vorteilhaft ist die Kombination mit anderen Verfahren zur Detektion von elektromagnetischen Feldern, speziell MRI-Feldem, und Kombination mit unterschiedlichen Reaktionen auf elektromagnetische Felder, speziell MRI-Felder. Als weitere Detektionsverfahren sind neben anderen auch:
- GMR-Sensoren,
- MagFETs,
- Auswertung von induzierten Strömen in Elektroden oder Antennen als Indikator,
- Detektion von spezifischen Vibrationen oder als Sensoren ausgelegte Bauteile zur Detektion von durch Lorentzkräfte induzierten Vibrationen als Indikator und
- Lagesensoren als Indikator vorteilhafte vorsehbar.

Unter Indikatoren sind dabei Verfahren und/oder Vorrichtungen zu verstehen, die Rückschlüsse auf das Vorhandensein von elektromagnetischen Störfeldern zulassen.

Als weitere Reaktionen auf detektierte elektromagnetische Felder, speziell MRI-Felder sind neben den bereits genannten auch anderen Reaktionen möglich, wie, aber nicht beschränkt auf,
- ein verlängertes Verbleiben in einem MRI-sicheren oder gegenüber elektromagnetischen Störfeldern unempfindlichen Zustand,
- eine Synchronisierung von elektrischen Messungen (z. B. Impedanzmessungen) mit bei periodischen oder gepulsten elektromagnetischen Feldern auftretenden betragsmäßigen Feldstärkenminima oder die Synchronisierung einer Stimulation mit eben jenen betragsmäßigen Minima und
- die Abgabe von elektromagnetischen Pulsen zur Signalisierung, dass ein medizinisches Gerät, speziell ein Implantat, im elektromagnetischen Feld vorhanden ist, speziell zur Signalisierung an ein MRI-Gerät, mit der Möglichkeit neben der Störung auch Informationen auf diese Weise zu übertragen und auf dem Bildschirm des MRI sichtbar zu machen.

Des Weiteren wird bevorzugt, dass ein Lagesensor zur Plausibilitätsprüfung herangezogen wird und eine positive MRI-Erkennung nur erfolgt, wenn der Lagesensor eine liegende Positur und/oder eine andere voreinstellbare Positur meldet.

Besonders bevorzugt ist der Lagesensor selbst kalibrierend, wobei die Kalibrierung unter voreinstellbaren Randbedingungen, wie aber nicht beschränkt auf, Uhrzeiten und/oder Herzraten und/oder Atemfrequenz und/oder hämodynamische Parameter und/oder Aktivität (Bewegungssensor) stattfindet.

Einige Aspekte der Erfindung werden in den Figuren 1 -4 dargestellt.
- Fig. 1: Schematische Darstellung des Ablaufs einer MRI-Untersuchung
- Fig. 2: Blockschaltbild eines erfindungsgemäßen IMD mit MRI-Erkennungseinheit
- Fig. 3A: Graphische Darstellung des typischen Batteriespannungsverlaufs über der Zeit beim Aufladen eines Schockkondensators
- Fig. 3B: Graphische Darstellung des typischen Batteriespannungsverlaufs über der Zeit beim Aufladen eines Schockkondensators über einen magnetisch vorgesättigten Transformatorkern
- Fig. 4: Graphische Darstellung des typischen Batteriespannungsverlaufs über der Zeit beim Aufladen eines Schockkondensators mit einer teilweise entladenen Batterie

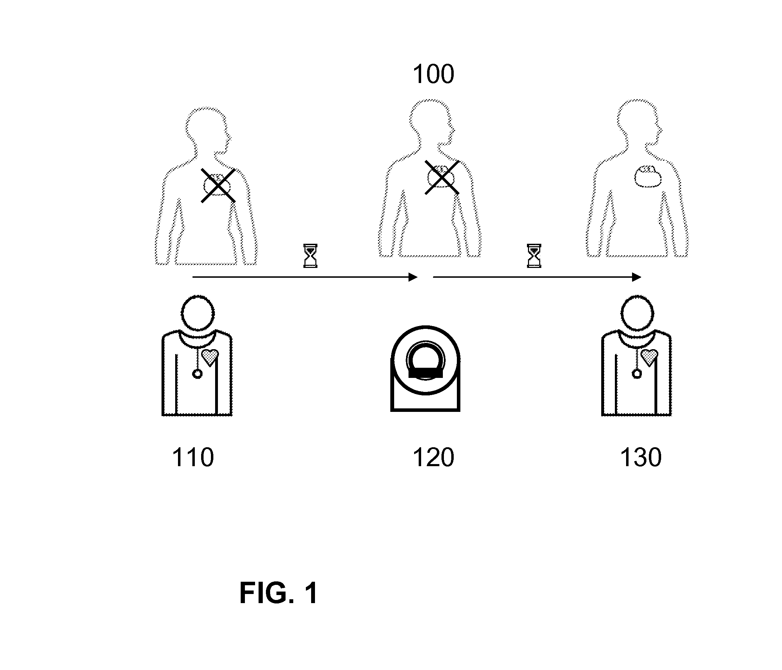

Figur 1 beschreibt den Stand der Technik, bei dem der ICD-Patient 100 vor der geplanten MRT-Untersuchung von einem Kardiologen nachgesorgt und der ICD ausgeschaltet 110 wird. Mit einer zeitlichen Verzögerung von Stunden bis Tagen erfolgt die MRT-Untersuchung bei einem Radiologen 120. Nach einer weiteren Verzögerung wird der Patient wieder vom Kardiologen 130 betreut und der ICD wieder eingeschaltet. Während der gesamten Zeit von 110 bis 130 ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung, in Kauf genommen.

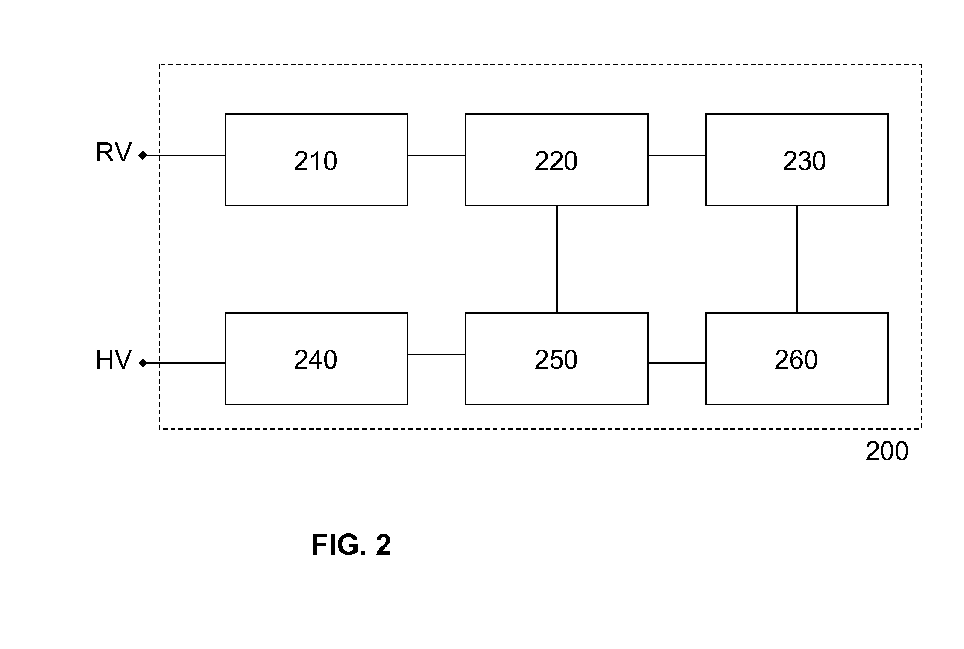

Die Figur 2 zeigt das Blockschaltbild der erfindungsgemäßen Lösung zur Detektion eines MRT-Magnetfeldes in einem ICD 200. Die zur Detektion von ventrikulärem Flimmern (VF) eingesetzte Elektrode, bevorzugt eine rechtsventrikuläre Elektrode (RV), ist mit einer Einheit zur EKG-Signalwahrnehmung/Rhythmusklassifikation und Stimulation 210 verbunden. Diese wiederum ist mit einer Kontrolleinheit verbunden, die bei einer VF-Detektion die Hochspannungsladeschaltung 250 zum Aufladen der Hochspannungskondensatoren in der Schocktherapieeinheit 240 startet. Diese Hochspannungsladeschaltung 250 umfasst dabei einen Transformator, der derart dimensioniert ist, dass dieser durch das Magnetfeld eines MRT ganz oder teilweise magnetisch gesättigt wird. Die Ladeschaltung wird durch eine Batterie 260 versorgt. Erfindungsgemäß ist diese Batterie mit einer Batteriespannungsüberwachungseinheit 230 verbunden, die so ausgeführt ist, dass das dynamische Batteriespannungsverhalten bei und nach Ladebeginn erfasst und mit Vergleichsparametem bewertet wird. Die Batteriespannungsüberwachungseinheit 230 ist ebenfalls mit der Steuereinheit 220 verbunden. Wird in der Batteriespannungsüberwachungseinheit 230 eine magnetische Vorsättigung des Transformators der Ladeschaltung 250 klassifiziert, so wird in der Steuereinheit der Ladevorgang abgebrochen und die VF-Detektion für eine programmierbare Zeit ausgesetzt.

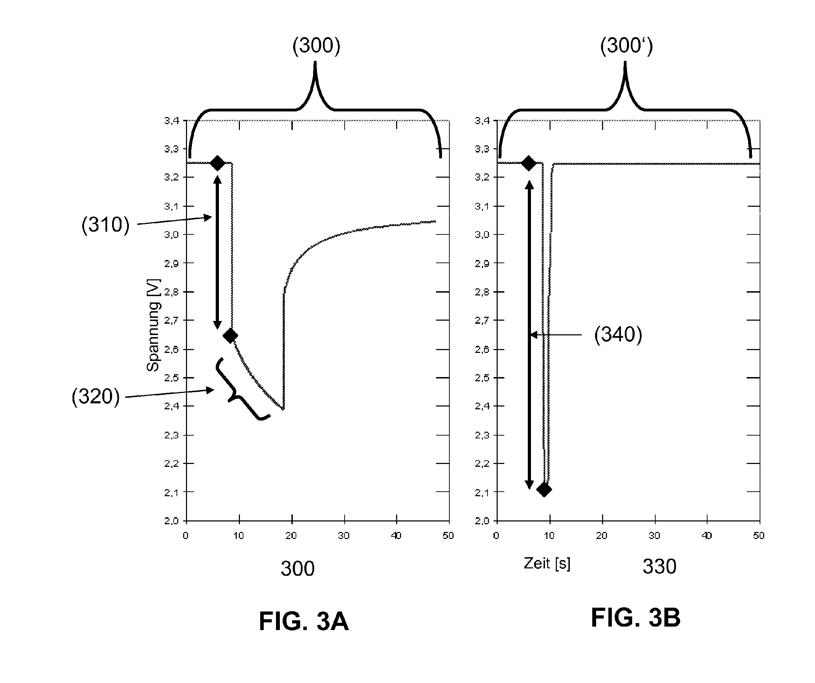

In der Figur 3a ist zunächst der typische Batteriespannungsverlauf bei einer Aufladung der Schockkondensatoren 300 dargestellt. Mit dem Einschalten der Hochspannungsladeschaltung bricht die Batteriespannung 310 zunächst schlagartig ein. Dieser Spannungseinbruch resultiert aus dem Batterieinnenwiderstand und dem fließenden Ladestrom. Während des Aufladens der Schockkondensatoren (typischerweise innerhalb von 5-10 Sekunden) sinkt dann die Batteriespannung 320 weiter ab. Dieses Absinken wird durch die elektrochemischen Systeme der Batterie dominiert.

Figur 3b zeigt den Spannungsverlauf bei stark vorgesättigtem Transformatorkern. Hier bricht mit dem Ladebeginn die Batteriespannung 340 viel weiter ein, da durch die Sättigung des Transformators dessen Induktivität herabgesetzt wird und damit der Ladestrom signifikant gegenüber einem normalen Ladezyklus ansteigt. Bei gleichem Batterieinnenwiderstand bricht die Spannung dementsprechend stärker ein.

In der einfachsten erfindungsgemäßen Implementierung vergleicht die Spannungsüberwachungseinheit 230 aus Abbildung 1 lediglich den Spannungseinbruch unmittelbar nach Ladebeginn mit einem programmierten Schwellwert. Wird dieser überschritten, so wird der Ladevorgang sofort beendet und ein Signal an eine Steuereinheit gesendet oder für eine programmierbare Zeit die VT/VF-Detektion gesperrt.

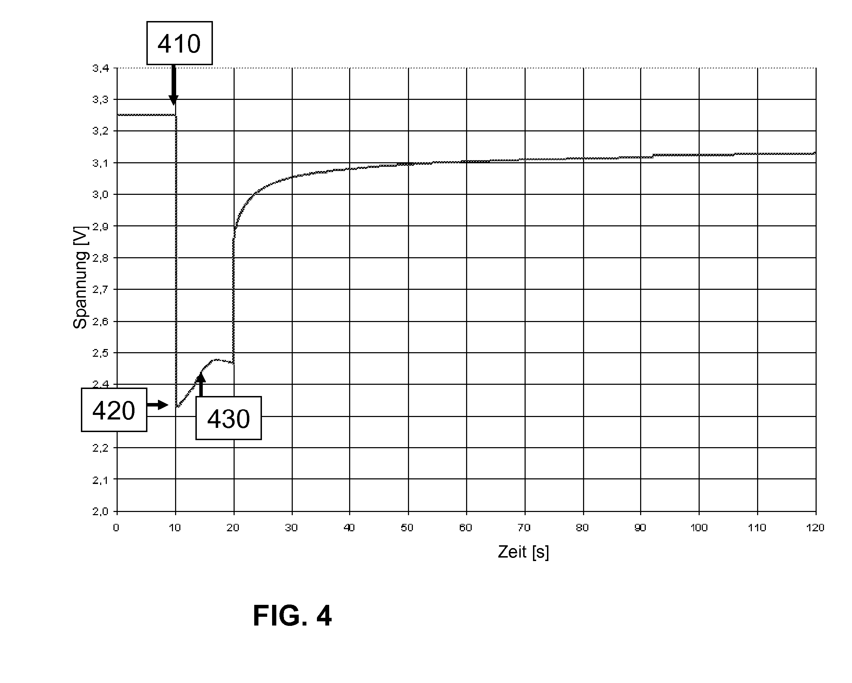

Figur 4 zeigt eine graphische Darstellung mit einer Batterie am Ende der Betriebszeit mit ausgeprägtem "Voltage Delay". Die Leerlaufspannung 410 ist unauffällig. Jedoch bricht die Batteriespannung beim Ladebeginn 420 übermäßig ein, was mit einem gesättigtem Trafo verwechselt werden kann. Um dies zu vermeiden erfolgt zu einem späteren Zeitpunkt eine weitere Spannungsmessung 430. Liegt diese Spannung über dem ersten Wert nach Ladebeginn 420, dann ist von einem Voltage Delay und nicht von einer Trafosättigung auszugehen.

Die zweite Messung ist vor Allem dann sinnvoll, wenn das Implantat mindestens die zweite Hälfte der erwarteten Betriebszeit erreicht hat und eine Silber Vanadiumoxid/SVO-Batterie besitzt.

## Patentansprüche

1. Batteriebetriebenes elektrisches Gerät und/oder implantierbares medizinisches Gerät **dadurch gekennzeichnet, dass** es mindestens beinhaltet:
eine Batteriespannungsüberwachungseinheit (230), die ausgestaltet ist einen Spannungsverlauf (300, 300') an einer Batterie während des Ladens eines Kondensators über einen Transformator in einer Ladeschaltung (250) zu überwachen und anhand des Spannungsverlaufs eine magnetische Vorsättigung des Transformators zu bestimmen und
eine Kontrolleinheit (220), die den Ladevorgang immer dann abbricht, wenn ein vordefinierter Schwellwert für die magnetische Vorsättigung des Transformators überschritten wird.

2. Batteriebetriebenes elektrisches Gerät nach Anspruch 1, **dadurch gekennzeichnet, dass** die Erkennung der Überschreitung des Schwellwerts für die magnetische Vorsättigung des Transformators über eine Messung einer minimalen Batteriespannung während des Ladens erfolgt.

3. Batteriebetriebenes elektrisches Gerät und/oder implantierbares medizinisches Gerät nach Anspruch 1, **dadurch gekennzeichnet, dass** die Erkennung der Überschreitung des Schwellwerts für die magnetische Vorsättigung des Transformators über eine Messung eines maximalen Spannungsgradienten dU/dt am Ladebeginn erfolgt.

4. Batteriebetriebenes elektrisches Gerät nach Anspruch 1, **dadurch gekennzeichnet, dass** die Erkennung einer Überschreitung der Schwelle für die magnetische Vorsättigung des Transformators über eine Messung einer maximalen Spannungsdifferenz vor und während des Ladens erfolgt.

5. Batteriebetriebenes elektrisches Gerät nach Anspruch 1, **dadurch gekennzeichnet, dass** die Erkennung einer Überschreitung der Schwelle für die magnetische Vorsättigung des Transformators über Messungen einer minimalen Batteriespannung während des Ladens und einer maximalen Spannungsdifferenz vor und während des Ladens erfolgt.

6. Batteriebetriebenes elektrisches Gerät nach einem der vorhergehenden Ansprüche, **dadurch gekennzeichnet, dass** der Ladestrom derart begrenzt wird, dass eine minimale Batteriespannung nicht unterschritten wird.

7. Batteriebetriebenes elektrisches Gerät nach einem der vorhergehenden Ansprüche, **dadurch gekennzeichnet, dass** bei Abbruch des Ladevorgangs die Ladeschaltung für eine vorgegebene Zeit deaktiviert wird.

8. Batteriebetriebenes elektrisches Gerät nach Anspruch 7, **dadurch gekennzeichnet, dass** die vorgegebene Zeit für die Deaktivierung der Ladeschaltung (250) bei aufeinander folgenden Ladeabbrüchen innerhalb einer zweiten vorgegebenen Zeit durch die Kontrolleinheit (220) und die Batteriespannungsüberwachungseinheit (230) jeweils um eine dritte vorgegebene Zeit verkürzt wird, wobei eine vorgegebene minimale Zeit für die Deaktivierung der Ladeschaltung nicht unterschritten wird und die erste Zeit zur Deaktivierung der Ladeschaltung (250) nach einer vierten vorgegebenen Zeit wieder hergestellt wird.

9. Batteriebetriebenes elektrisches Gerät nach einem der vorhergehenden Ansprüche, **dadurch gekennzeichnet, dass** das Gerät durch Programmierung und/oder durch Femprogrammierung, "remote-programming", in einen MRI-sicheren Zustand versetzbar ist.

10. Batteriebetriebenes elektrisches Gerät nach Anspruch 9, **dadurch gekennzeichnet, dass** die Kontrolleinheit (220) den Ladevorgang bei Erreichen oder Überschreiten des Schwellwertes für die Vorsättigung des Transformators abbricht und das batteriebetriebene elektrische Gerät für einen vorbestimmbaren Zeitraum in den MRI-sicheren Zustand versetzt.

11. Batteriebetriebenes elektrisches Gerät nach einem der Ansprüche 9 bis 10, **dadurch gekennzeichnet, dass** ein Einschalten, oder das Eingeschaltet sein des MRI-sicheren Zustands mittels telemetrischer Fernüberwachung übertragen wird.

12. Batteriebetriebenes elektrisches Gerät nach einem der vorhergehenden Ansprüche **dadurch gekennzeichnet, dass** das batteriebetriebene elektrische Gerät ein implantierbares medizinisches Gerät (IMD) ist.

13. Batteriebetriebenes elektrisches Gerät nach Anspruch 12. **dadurch gekennzeichnet, dass** das IMD ein implantierbarer Defibrillator/Kardioverter (ICD) oder ein IMD zur kardialen Resynchronisation mit einem ICD ist.

14. Batteriebetriebenes elektrisches Gerät nach Anspruch 13, **dadurch gekennzeichnet, dass** ein MRI-sicherer Zustand das Unterdrücken der Abgabe von Hochspannungsschocks umfasst.

15. Batteriebetriebenes elektrisches Gerät nach Anspruch 13 oder 14, **dadurch gekennzeichnet, dass** ein MRI-sicherer Zustand ein Unterdrücken der Abgabe von Schrittmacherstimulationen enthält oder aber eine Abgabe von asynchronen Schrittmacherimpulsen umfasst.
